# EUROPÄISCHE PATENTSCHRIFT

(11) **EP 0 585 680 B1**
(45) Veröffentlichungstag und Bekanntmachung des Hinweises auf die Patenterteilung: **28.02.1996**
(21) Anmeldenummer: 93112922.5
(22) Anmeldetag: 12.08.1993
(51) Int. Cl.: C07C 29/56, C07C 33/03

(54) **Verfahren zur katalytischen Isomerisierung von alpha-Alkenolen unter Verwendung von organischen Derivaten von Rheniumoxiden**
Catalytic isomerization of alpha alkenols using organic derivatives of rhenium oxides
Procédé pour l'isomérisation catalytique d'alpha alcénols utilisant des dérivés organiques d'oxydes de rhénium

(30) Priorität: 29.08.1992 DE 4228887
(43) Veröffentlichungstag der Anmeldung: 09.03.1994
(73) Patentinhaber: BASF Aktiengesellschaft, D-67063 Ludwigshafen (DE)
(72) Erfinder: Thome, Alfred, Dr., D-6700 Ludwigshafen (DE); Roeper, Michael, Prof. Dr., D-6706 Wachenheim (DE); Kneuper, Heinz-Josef, Dr., D-6800 Mannheim 1 (DE)

(56) Entgegenhaltungen:
- EP-A- 0 373 488
- DE-A- 2 752 986
- US-A- 3 925 485
- US-A- 4 006 193

## Beschreibung

Die vorliegende Erfindung betrifft ein Verfahren zur katalytischen Isomerisierung von sekundären oder tertiären α-Alkenolen der Formel I 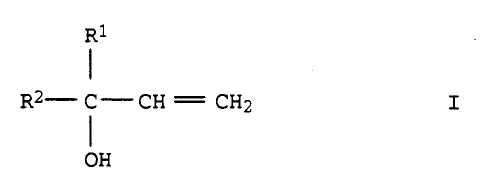
in der R¹ eine C₁- bis C₂₀-Alkyl- oder eine C₂- bis C₂₀-Alkenylgruppe ist und in der R² für Wasserstoff, ein Halogenatom, eine C₁- bis C₁₀-Alkoxygruppe, eine Carbonylgruppe oder eine C₁- bis C₂₀-Alkylgruppe steht und in der R¹ und R² gemeinsam einen 5- bis 6-gliedrigen, carbocyclischen Ring bilden können, in primäre α-Alkenole der Formel II 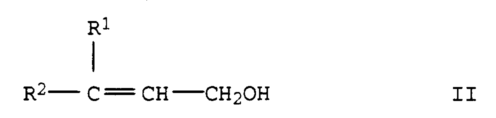
oder zur Isomerisierung von primären α-Alkenolen der Formel II in sekundäre α-Alkenole der Formel I.

Die Isomerisierung der Allylalkohole I in die entsprechenden Allylalkohole II und umgekehrt, ist eine Gleichgewichtsreaktion. Sie kann durch verschiedenerlei Katalysatoren, z.B. durch Brönstedt-Säuren (vgl. J. Am. Chem. Soc. 61, 2564 (1939)) oder Übergangsmetallverbindungen katalysiert werden. Da die Isomerisierung mittels Brönstedt-Säuren je nach Substrat zu einer Vielzahl unerwünschter Nebenreaktionen führen kann, wurde schon früh versucht, Übergangsmetallverbindungen zu finden, die diese Umsetzung selektiver katalysieren.

In DE-A-27 52 986 wird die Isomerisierung von Allylalkoholen mit Hilfe von Palladiumkomplexen beschrieben. Aufgrund des hohen Preises und des Umstandes, daß Palladiumkomplexe chemisch nicht sehr stabil sind und unter den Reaktions- und Aufarbeitungsbedingungen zur Abscheidung von fein verteiltem, metallischem Palladium an den Wandungen der Reaktionsapparaturen neigen, ist diese Methode für industrielle Zwecke nicht geeignet.

In US-A 4 006 193 werden eine Reihe von Verbindungen von Übergangsmetallen der Nebengruppen V, VI und VII des Periodensystems zur Isomerisierung von Allylalkoholen verwendet. Obwohl diese Schrift eine Vielzahl von Übergangsmetallverbindungen als Katalysatoren für diese Isomerisierung als geeignet bezeichnet, werden danach Vanadiumverbindungen, insbesondere Vanadate und Vanadatester mit aliphatischen Alkoholen bevorzugt als Katalysatoren zur Isomerisierung verwendet, da mit diesen Verbindungen die besten Ergebnisse bezüglich Umsatz und Selektivität erzielt werden. Rheniumverbindungen, insbesondere die Ester, Anhydride und Ammoniumsalze der Perrheniumsäure, werden in dieser Schrift ebenfalls als geeignete Katalysatoren angeführt, das einzige Beispiel einer Isomerisierung mittels einer Rheniumverbindung, nämlich mittels Ammoniumperrhenat, ergibt jedoch einen deutlich schlechteren Umsatz und eine deutlich schlechtere Selektivität als die analoge Umsetzung mit Vanadiumverbindungen.

Auch gemäß US-A 3 925 485 werden mit Vanadiumverbindungen die besten Ergebnisse bei der Isomerisierung von primären und sekundären Allylalkoholen erzielt. Auch in dieser Schrift werden eine Reihe von Rheniumverbindungen als Isomerisierungskatalysatoren genannt, ein Beispiel zur Isomerisierung von Allylalkoholen mittels einer dieser Rheniumverbindungen wird in dieser Schrift allerdings nicht gegeben.

Obwohl mit Katalysatoren aus Vanadiumverbindungen gemäß US-A 4 006 193 und US-A 3 925 485 im Vergleich zu Katalysatoren aus Verbindungen der übrigen Übergangsmetalle der V., VI. und VII. Nebengruppe des Periodensystems die besten Ergebnisse bei der Isomerisierung von Allylalkoholen erzielt werden, führt auch die Verwendung von Vanadiumverbindungen nur zu unbefriedigenden Ergebnissen hinsichtlich der Selektivität der Isomerisierungsreaktion, so daß die Anwendung der Verfahren dieser beiden Patente, selbst in ihren am meisten bevorzugten Ausgestaltungen, auf die Herstellung von Massenchemikalien, z.B. von Vorprodukten für Weichmacheralkohole, infolge des hohen Anfalls nicht verwertbarer Nebenprodukte, nicht wirtschaftlich ist.

Bei der Telomerisation von Butadien mit Wasser (vgl. z.B. US-A 4 962 243) gemäß Gleichung (1) 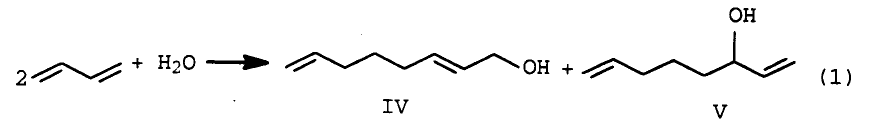
und bei der Wasseraddition an Butadien (vgl. z.B. US-A 4 645 863) gemäß Gleichung (2) 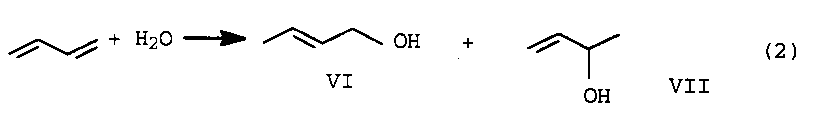
entsteht in erheblichem Maße das unerwünschte sekundäre Octadienol V bzw. das unerwünschte sekundäre Butenol VII als Nebenprodukt. Da aber nur die primären Alkenole IV und VI zu den als Weichmacheralkoholen bzw. als Lösungsmittel dienenden Alkanolen, n-Octanol bzw. n-Butanol hydriert werden können, wird ein auch im großtechnischen Maßstab bei hohen Umsätzen mit guter Selektivität durchführbares Verfahren zur Isomerisierung von Allylalkoholen gesucht.

Der vorliegenden Erfindung lag deshalb die Aufgabe zugrunde, ein Verfahren zur Isomerisierung von primären oder sekundären Allylalkoholen zu finden, das diese Isomerisierung mit hohen Umsätzen und einer hohen Selektivität ermöglicht und so die Anwendung dieser Umsetzung vor allem auch für großtechnische Verfahren, beispielsweise die Isomerisierung des sekundären Octadienols V, möglich und wirtschaftlich macht. Insbesondere sollten dazu wirksamere und selektivere Katalysatoren als die bisher bekannten sowie geeignete Aufarbeitungsmethoden gefunden werden, die zu einer geringeren Bildung unerwünschter Nebenprodukte führen.

Dementsprechend wurde ein Verfahren zur katalytischen Isomerisierung von sekundären oder tertiären α-Alkenolen der Formel I 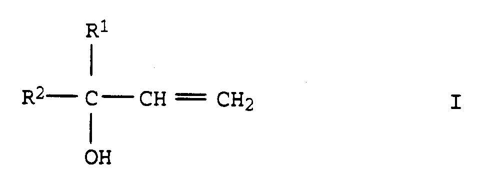
in der R¹ eine C₁- bis C₂₀-Alkyl- oder eine C₂- bis C₂₀-Alkenylgruppe ist und in der R² für Wasserstoff, ein Halogenatom, eine C₁- bis C₁₀-Alkoxygruppe, eine Carbonylgruppe oder eine C₁- bis C₂₀-Alkylgruppe steht und in der R¹ und R² gemeinsam einen 5- bis 6-gliedrigen, carbocyclischen Ring bilden können, in primäre α-Alkenole der Formel II 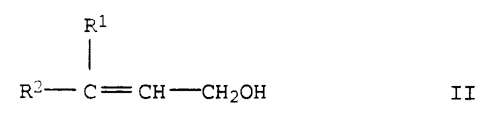
oder zur Isomerisierung von primären α-Alkenolen der Formel II in sekundäre oder tertiäre α-Alkenole der Formel I gefunden, das dadurch gekennzeichnet ist, daß man die Isomerisierung in Gegenwart von Organotrioxorhenium(VII)-Verbindungen der Formel III 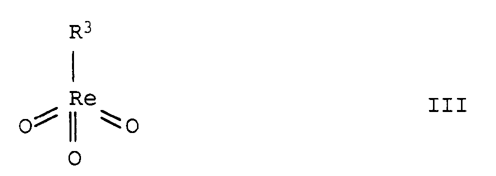
vornimmt, in der R³ eine C₁- bis C₁₀-Alkylgruppe, eine unsubstituierte oder eine mit 1 bis 5 C₁- bis C₄-Alkylgruppen substituierte Cyclopentadienylgruppe, eine C₆- bis C₁₀-Aryl- oder eine C₇- bis C₁₁-Aralkylgruppe ist oder daß man die Isomerisierung in Gegenwart von an ein polymeres Trägermaterial fixierten Organotrioxorhenium(VII)-Verbindungen (III) ausführt.

Bei den erfindungsgemäß zu verwendenden Isomerisierungskatalysatoren III handelt es sich um eine relativ neue Klasse von Rheniumverbindungen, deren erste Vertreter Mitte der 80er Jahre erstmals hergestellt werden konnten. Methyltrioxorhenium(VII) (R³=CH₃) wurde z.B. erstmals 1988 durch die Behandlung von Dirheniumheptoxid (Re₂O₇) mit Tetramethylzinn erhalten (Angew. Chem. 100, 420 (1988)). Höhere Alkyl- und Aryl-trioxorhenium(VII)-Verbindungen sind seit neuestem durch die Umsetzung von Dirheniumheptoxid mit Dialkylzink-Verbindungen gemäß Angew. Chem. 103, 183 (1991) erhältlich und Cyclopentadienyltrioxorhenium(VII)-Derivate können durch die Oxidation von Cyclopentadienyltricarbonylrheniumverbindungen mit Wasserstoffperoxid hergestellt werden (J. Organomet. Chem. 297, C5 (1985)). Übersichtsartikel über diese Rheniumverbindungen und deren Herstellung befinden sich in Angew. Chem. 100, 1269 (1988) und in J. Organomet. Chem. 382, 1 (1990).

Bevorzugt werden im erfindungsgemäßen Verfahren solche Organotrioxorhenium(VII)-Verbindungen III verwendet, in denen R³ eine C₁- bis C₅-Alkylgruppe, die Pentamethylcyclopentadienyl- oder die Phenylgruppe ist. Besonders bevorzugt werden Methyltrioxorhenium(VII) und Pentamethylcyclopentadienyltrioxorhenium(VII) eingesetzt.

Die Rheniumkatalysatoren III können in Substanz oder in Lösung erfindungsgemäß eingesetzt werden. In einer anderen Ausgestaltung des erfindungsgemäßen Verfahrens werden die Rheniumverbindungen III an einem polymeren oder mineralischen Träger fixiert in die Umsetzung eingebracht. Als polymere Trägermaterialien werden für diesen Zweck vorzugsweise polymere Trägermaterialien verwendet, die an ihrer Oberfläche stickstoffhaltige Gruppen tragen, wobei die Stickstoffatome sowohl in aromatische als auch in aliphatische Gruppen und darüber hinaus in Amid- oder Carbamatgruppen eingebaut sein können. Beispielhaft für solche stickstoffhaltigen Polymere seien Polyvinylpyrrolidon, Poly(2-vinylpyridin), (2-Vinylpyridin-)Styrol-Copolymere, Polyacrylsäureamide, Polyimide, Polyamide und Polyurethane genannt. Diese stickstoffhaltigen Polymere fixieren die Verbindungen III durch eine koordinative Bindung, so daß man diese Substanzen als Koordinationsverbindungen der Formel VIII

(Polymer)ₐ · (R³ReO₃)_{b} VIII

formulieren kann, worin der Quotient b/a das Gewichtsverhältnis der beiden Bestandteile darstellt, welches im allgemeinen 0,01 bis 0,2, vorzugsweise 0,02 bis 0,1 beträgt. Im allgemeinen gilt dabei die Regel, daß mit der Erhöhung des Rheniumgehalts von VIII die Reaktionsgeschwindigkeit der Isomerisierungsreaktion beim Einsatz aliquoter Mengen des Katalysators VIII zunimmt. Die Verwendung derartiger polymerer Rheniumkatalysatoren VIII hat den Vorteil, daß der Katalysator nach erfolgter Umsetzung auf einfache Weise, z.B. durch Filtration vom Reaktionsgemisch abgetrennt werden kann. Zur Herstellung derartiger polymerer Katalysatoren VIII bedient man sich zweckmäßigerweise eines Imprägnierverfahrens, bei dem das betreffende Polymer einmal oder mehrfach mit einer Lösung des jeweiligen Rheniumkatalysators III in einem geeigneten Lösungsmittel, z.B. einem Ether, wie Dimethoxyethan, Tetrahydrofuran oder Dioxan, getränkt und anschließend getrocknet wird. Bezüglich weiterer Einzelheiten zur Herstellung polymerer Katalysatoren VIII verweisen wir auf DE-A 39 02 357.

Im allgemeinen werden die Katalysatoren der Formel III im erfindungsgemäßen Verfahren in Mengen eingesetzt, die einem Rhenium (berechnet als Re)/α-Alkenol-Molverhältnis von 1:50 bis 1:5000, vorzugsweise von 1:75 bis 1:2500 und besonders bevorzugt von 1:100 bis 1:1000 entsprechen. Bei Verwendung von an Polymere fixierten Rheniumkatalysatoren der Formel VIII werden diese dem zu isomerisierenden Allylalkohol in solchen Mengen zugesetzt, daß das Rhenium(berechnet als Re)/Allylalkohol-Molverhältnis im Reaktionsgemisch im allgemeinen 1:50 bis 1:5000, vorzugsweise 1:75 bis 1:2500 und besonders bevorzugt 1:100 bis 1:1000 beträgt. Der Rheniumgehalt des Polymeren kann dabei nach dem Verfahren der Röntgenfluoreszenz- oder Atomemissionsspektrometrie bestimmt werden.

Das erfindungsgemäße Verfahren kann in Gegenwart von Luft durchgeführt werden. Vorzugsweise wird aber in einer Inertgasatmosphäre, z.B. in einer Stickstoff- oder Argonatmosphäre, gearbeitet.

Zweckmäßigerweise wird das erfindungsgemäße Verfahren bei Temperaturen von 0 bis 300°C, vornehmlich bei 10 bis 200°C und besonders bevorzugt bei 20 bis 150°C, betrieben. Bezüglich des Reaktionsdrucks unterliegt das erfindungsgemäße Verfahren keinen Beschränkungen und kann bei Atmosphärendruck, erhöhtem oder vermindertem Druck gleich gut ausgeübt werden. Im Hinblick auf die Aufarbeitung des Reaktionsgemisches, beispielsweise im Falle der kontinuierlichen, destillativen Ausschleusung eines Isomerisierungsproduktes aus der Reaktion, kann es zweckmäßig sein, einen an die Durchführung dieser Destillation optimal angepaßten Druck in der Reaktionsapparatur einzustellen.

Das erfindungsgemäße Verfahren kann sowohl in An- als auch in Abwesenheit von Lösungsmitteln ausgeführt werden. Zweckmäßigerweise verwendet man solche Lösungsmittel, die unter den Reaktionsbedingungen chemisch stabil sind und die Leistung der eingesetzten Katalysatoren nicht beeinträchtigen. Als Lösungsmittel werden zweckmäßigerweise hochsiedende Lösungsmittel, z.B. Ether, wie Polyethylenglykolether oder Polypropylenglykolether, Di- und Triethylenglykoldimethylether oder hochsiedende Alkanole wie Palmityl-, Stearyl- oder Cetylalkohol sowie Di- und Triethylenglykol, hochsiedende, halogenierte, vorzugsweise chlorierte Kohlenwasserstoffe, wie Chlorbenzol, Dichlorbenzol und dgl., Carbonsäureamide, wie N,N-Dimethylformamid, Sulfoxide, wie Dimethylsulfoxid und Sulfolan oder hochsiedende Kohlenwasserstoffe, wie Xylol, Squalan oder Paraffinöle. Die Menge des verwendeten Lösungsmittels ist, wie erwähnt, in der Regel nicht kritisch und kann in weiten Bereichen variiert werden. Im allgemeinen wird das Lösungsmittel in Mengen von 5 bis 100 Gew.-%, bezogen auf das Gewicht der Reaktionsmischung ohne Lösungsmittel, zugesetzt.

Da die Isomerisierung der sekundären oder tertiären zu den primären α-Alkenolen und umgekehrt gemäß Gleichung (3) 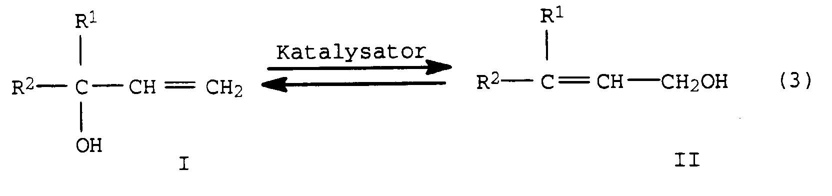
eine Gleichgewichtsreaktion ist, kommt die Isomerisierungsreaktion nach Einstellung der Gleichsgewichtsbedingungen zum Stillstand und man erhält als Produkt eine Mischung der Allylalkohole I und II. Ist der Allylalkohol I das gewünschte Produkt der Isomerisierung, so kann er aufgrund seines im Vergleich zu II niedrigeren Siedepunktes aus der Reaktionsmischung kontinuierlich, wie er entsteht, abdestilliert und mit Hilfe dieser Maßnahme, der primäre Allylalkohol II vollständig in den sekundären oder tertiären Allylalkohol I umgesetzt werden.

Ist hingegen der primäre Allylalkohol II das gewünschte Produkt der Isomerisierung, wird zu dessen Gewinnung zweckmäßigerweise so verfahren, daß die Reaktionsmischung nach Einstellung des Gleichsgewichts destillativ in die Allylalkohole I und II aufgetrennt und der sekundäre Allylalkohol I wieder in die Umsetzung zurückgeführt wird. Bei dieser Ausgestaltung des erfindungsgemäßen Verfahrens ist die Verwendung eines hochsiedenden Lösungsmittels vorteilhaft. Der Katalysator bleibt während der Destillation im Lösungsmittel gelöst.

Bezüglich der Anwendbarkeit des erfindungsgemäßen Verfahrens zur Isomerisierung von primären, sekundären oder tertiären Allylalkoholen der Formeln I oder II sind keine Beschränkungen bekannt. Beispielsweise kann der Rest R¹ dieser Allylalkohole für eine C₁- bis C₂₀-Alkyl-, vorzugsweise eine C₁- bis C₁₀- und besonders bevorzugt eine C₁- bis C₅-Alkylgruppe oder eine C₂- bis C₂₀-, vorzugsweise eine C₂- bis C₁₀-, insbesondere eines C₂- bis C₈-Alkenylgruppe mit 1 oder 2 Doppelbindungen, vorzugsweise mit einer Doppelbindung, sein. Der Rest R² der Allylalkohole I und II kann z.B. für Wasserstoff stehen oder eine C₁- bis C₂₀-, vorzugsweise eine C₁- bis C₁₀-Alkylgruppe bedeuten. Des weiteren kann R² für eine C₁- bis C₁₀-, vorzugsweise eine C₁- bis C₄-Alkoxygruppe, für ein Halogenatom, insbesondere für ein Chloratom oder eine Carbonylgruppe stehen. Die Reste R¹ und R² können auch gemeinsam einen fünf- bis sechsgliedrigen, carbocyclischen Ring bilden, der 1 bis 2 Doppelbindungen, vorzugsweise nicht mehr als eine Doppelbindung, enthalten kann oder gesättigt ist. Die Reste R¹ und/oder R² können unsubstituiert sein oder 1 bis 3, vorzugsweise einen Substituenten tragen, die z.B. ein Halogenatom, insbesondere ein Chloratom, eine C₁- bis C₄-Alkoxygruppe, eine Carbonyl- oder Oxogruppe sein können.

Beispielhaft für solche Allylalkohole, die nach dem erfindungsgemäßen Verfahren isomerisiert werden können, seien die folgenden α-Alkenole aufgezählt:
2-Methyl-3-buten-2-ol, 1,7-Octadien-3-ol, Linalool (3,7-Dimethyl-1,6-octadien-3-ol), 1-Vinyl-cyclohexanol, 1-Hexen-3-ol, Isophytol (3,7,11,15-Tetramethyl-1-hexadecen-3-ol), 2,3-Dimethyl-3-buten-2-ol, 1-Buten-3-ol, für α-Alkenole der Formel I, sowie
2-Buten-1-ol (Crotylalkohol), 3-Methyl-2-buten-1-ol (Prenylalkohol), 2,3-Dimethyl-2-buten-1-ol, 2-Hexen-1-ol, 2,7-Octadien-1-ol, Geraniol (3,7-Dimethyl-2,6-octadien-1-ol), Farnesol (3,7,11-Trimethyl-2,6,10-dodecatrien-1-ol), Phytol (3,7,11,15-Tetramethyl-2-hexadecen-1-ol), Vitamin A-Alkohol (Retinol), 2-Hexen-1-ol, 2,3-Dimethyl-2-buten-1-ol für α-Alkenole der Formel II.

Besonders bevorzugt wird das erfindungsgemäße Verfahren zur Isomerisierung von 1,7-Octadien-3-ol V zu 2,7-Octadien-1-ol IV sowie zur Isomerisierung von But-1-en-3-ol VII in But-2-en-1-ol VI benutzt.

### Beispiel

Zu 39,5 mg (0,16 mmol) Methyltrioxorhenium(VII) in 40 g Xylol, wurden unter Rühren bei einer Temperatur von 60°C 3 g (23,8 mmol) 1,7-Octadien-3-ol in 10 ml Xylol getropft. Die Umsetzung erfolgte unter einer Argonatmosphäre. Nach einer Reaktionszeit von 65 Minuten hatte sich das Gleichgewicht bei einem Umsatz von 36 % eingestellt. Die Selektivität für die 2,7-Octadien-1-ol-Bildung betrug 100 %.

## Patentansprüche

1. Verfahren zur katalytischen Isomerisierung von sekundären oder tertiären α-Alkenolen der Formel I 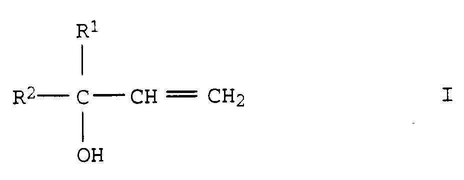 in der R¹ eine C₁- bis C₂₀-Alkyl- oder eine C₂- bis C₂₀-Alkenylgruppe ist und in der R² für Wasserstoff, ein Halogenatom, eine C₁- bis C₁₀-Alkoxygruppe, eine Carbonylgruppe oder eine C₁- bis C₂₀-Alkylgruppe steht und in der R¹ und R² gemeinsam einen 5- bis 6-gliedrigen, carbocyclischen Ring bilden können, in primäre α-Alkenole der Formel II 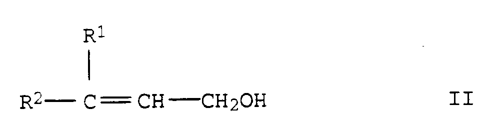 oder zur Isomerisierung von primären α-Alkenolen der Formel II in sekundäre oder tertiäre α-Alkenole der Formel I, dadurch gekennzeichnet, daß man die Isomerisierung in Gegenwart von Organotrioxorhenium(VII)-Verbindungen der Formel III 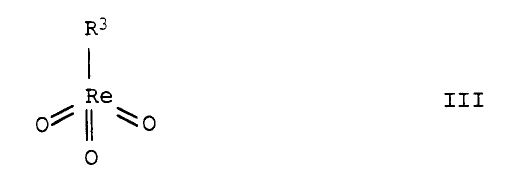 vornimmt, in der R³ eine C₁- bis C₁₀-Alkylgruppe, eine unsubstituierte oder eine mit 1 bis 5 C₁- bis C₄-Alkylgruppen substituierte Cyclopentadienylgruppe, eine C₆- bis C₁₀-Aryl- oder eine C₇- bis C₁₁-Aralkylgruppe ist oder daß man die Isomerisierung in Gegenwart von an ein polymeres Trägermaterial fixierten Organotrioxorhenium(VII)-Verbindungen (III) ausführt.

2. Verfahren gemäß Anspruch 1, dadurch gekennzeichnet, daß man die Isomerisierung in Gegenwart einer an ein stickstoffhaltiges Polymer fixierten Organotrioxorhenium(VII)-Verbindung der Formel VIII
(Polymer)ₐ · (R³ReO₃)_{b} VIII
durchführt, in der R³ die oben genannte Bedeutung hat und in der das Gewichtsverhältnis b/a 0,01 bis 0,2 beträgt.

3. Verfahren gemäß Anspruch 1, dadurch gekennzeichnet, daß man das α-Alkenol in Gegenwart einer Menge des Katalysators III umsetzt, die einem Rhenium/α-Alkenol-Molverhältnis von 1:50 bis 1:5000 entspricht.

4. Verfahren gemäß Anspruch 2, dadurch gekennzeichnet, daß man das α-Alkenol in Gegenwart einer Menge des Katalysators VIII umsetzt, die einem Rhenium/α-Alkenol-Molverhältnis von 1:50 bis 1:5000 entspricht.

5. Verfahren gemäß Anspruch 1, dadurch gekennzeichnet, daß man α-Alkenole der Formel I durch die Isomerisierung der α-Alkenole der Formel II erzeugt und dabei die α-Alkenole der Formel I, wie sie entstehen, kontinuierlich aus dem Isomerisierungsgemisch abdestilliert.

6. Verfahren gemäß Anspruch 1, dadurch gekennzeichnet, daß man α-Alkenole der Formel II durch die Isomerisierung der α-Alkenole der Formel I erzeugt, und das erhaltene Isomerisierungsgemisch in Gegenwart eines hochsiedenden Lösungsmittels destillativ auftrennt und das α-Alkenol der Formel I wieder in die Umsetzung zurückführt.

7. Verfahren gemäß Anspruch 1, dadurch gekennzeichnet, daß man es bei Temperaturen von 0 bis 300°C ausführt.

8. Verfahren gemäß Anspruch 1, dadurch gekennzeichnet, daß man 1,7-Octadien-3-ol zu 2,7-Octadien-1-ol oder But-1-en-3-ol zu But-2-en-1-ol isomerisiert.

## Claims

1. A process for catalytically isomerizing secondary or tertiary α-alkenols of the formula I 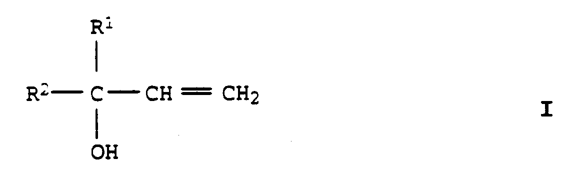 where R¹ is a C₁- to C₂₀-alkyl or a C₂- to C₂₀-alkenyl group and where R² is hydrogen, a halogen atom, a C₁- to C₁₀-alkoxy group, a carbonyl group or a C₁- to C₂₀-alkyl group and where R¹ and R² together can form a 5- to 6-membered carbocyclic ring, to primary α-alkenols of the formula II 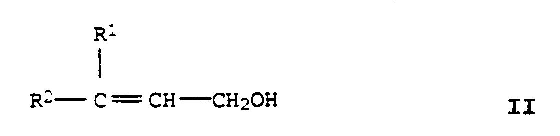 or for isomerizing primary α-alkenols of the formula II to secondary or tertiary α-alkenols of the formula I, which comprises performing the isomerization in the presence of organotrioxorhenium(VII) compounds of the formula III 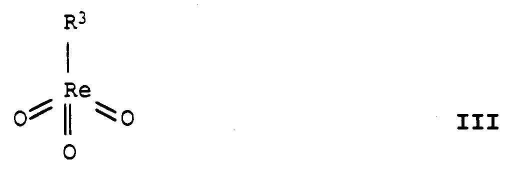 where R³ is a C₁- to C₁₀-alkyl group, an unsubstituted cyclopentadienyl group or one substituted by 1 to 5 C₁- to C₄-alkyl groups, a C₆- to C₁₀-aryl group or a C₇- to C₁₁-aralkyl group, or by carrying out the isomerization in the presence of organotrioxorhenium(VII) compounds (III) attached to a polymeric support material.

2. A process as claimed in claim 1, wherein the isomerization is carried out in the presences of an organotrioxorhenium(VII) compound attached to a nitrogen-containing polymer, of the formula VIII
(Polymer)ₐ · (R³ReO₃)_{b} VIII
where R³ has the abovementioned meanings and where the weight ratio b/a is from 0.01 to 0.2.

3. A process as claimed in claim 1, wherein the α-alkenol is reacted in the presence of an amount of the catalyst III which corresponds to a rhenium/α-alkenol molar ratio of from 1:50 to 1:5000.

4. A process as claimed in claim 2, wherein the α-alkenol is reacted in the presence of an amount of the catalyst VIII which corresponds to a rhenium/α-alkenol molar ratio of from 1:50 to 1:5000.

5. A process as claimed in claim 1, wherein α-alkenols of the formula I are produced by isomerizing the α-alkenols of the formula II and at the same time distilling off the α-alkenols of the formula I continuously from the isomerization mixture as they are formed.

6. A process as claimed in claim 1, wherein α-alkenols of the formula II are produced by isomerizing the α-alkenols of the formula I, and the isomerization mixture obtained is separated by distillation in the presence of a high-boiling solvent and the α-alkenol of the formula I is fed back into the reaction again.

7. A process as claimed in claim 1, which is carried out at from 0 to 300°C.

8. A process as claimed in claim 1, wherein 1,7-octadien-3-ol is isomerized to 2,7-octadien-1-ol or but-1-en-3-ol is isomerized to but-2-en-1-ol.

## Revendications

1. Procédé d'isomérisation catalytique d'α-alcénols secondaires ou tertiaires de la formule I 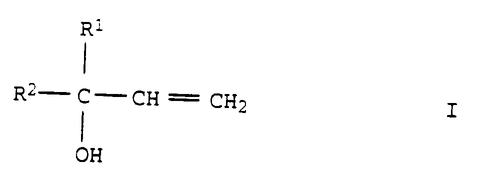 dans laquelle R¹ représente un radical alkyle en C₁ à C₂₀ ou un radical alcényle en C₂ à C₂₀ et dans laquelle R² représente un atome d'hydrogène, un atome d'halogène, un radical alcoxy en C₁ à C₁₀, un radical carbonyle, ou un radical alkyle en C₁ à C₂₀ et dans laquelle R¹ et R² peuvent former ensemble un noyau carbocyclique pentagonal ou hexagonal en α-alcénols primaires de la formule II 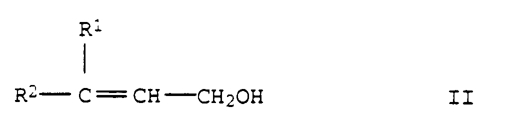 ou d'isomérisation d'α-alcénols primaires de la formule II en α-alcénols secondaires ou tertiaires de la formule I, caractérisé en ce que l'on entreprend l'isomérisation en présence de composés d'organotrioxorhénium (VII) de la formule III 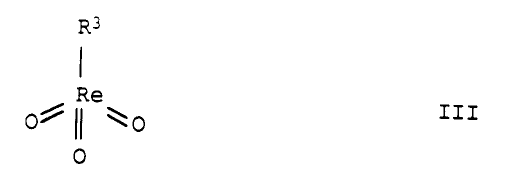 dans laquelle R³ représente un radical alkyle en C₁ à C₁₀, un radical cyclopentadiényle non substitué ou substitué par 1 à 5 radicaux alkyle en C₁ à C₄, un radical aryle en C₆ à C₁₀ ou un radical aralkyle en C₇ à C₁₁, ou en ce que l'on entreprend l'isomérisation en présence de composés (III) d'organotrioxorhénium (VII) fixés sur un support polymérique.

2. Procédé suivant la revendication 1, caractérisé en ce que l'on entreprend l'isomérisation en présence d'un composé d'organotrioxorhénium (VII) fixé sur un polymère contenant de l'azote, de la formule VIII
(Polymère)ₐ · (R³ReO₃)_{b} VIII
dans laquelle R³ possède les significations qui lui ont été attribuées ci-dessus et en ce que le rapport pondéral b/a varie de 0,01 à 0,2.

3. Procédé suivant la revendication 1, caractérisé en ce que l'on fait réagir l'α-alcénol en présence d'une proportion du catalyseur III qui correspond à un rapport molaire rhénium/α-alcénol de 1:50 à 1:5000.

4. Procédé suivant la revendication 2, caractérisé en ce que l'on fait réagir l'α-alcénol en présence d'une proportion du catalyseur VIII qui correspond à un rapport molaire rhénium/α-alcénol de 1:50 à 1:5000.

5. Procédé suivant la revendication 1, caractérisé en ce que l'on obtient des α-alcénols de la formule I par l'isomérisation des α-alcénols de la formule II et, ce faisant, on chasse les α-alcénols de la formule I en continu du mélange d'isomérisation, au fur et à mesure de leur formation.

6. Procédé suivant la revendication 1, caractérisé en ce que l'on obtient des α-alcénols de la formule II par l'isomérisation des α-alcénols de la formule I et en ce que l'on sépare le mélange d'isomérisation obtenu par distillation en présence d'un solvant à point d'ébullition élevé et on renvoie l'α-alcénol de la formule I à la réaction.

7. Procédé suivant la revendication 1, caractérisé en ce que l'on travaille à des températures de 0 à 300°C.

8. Procédé suivant la revendication 1, caractérisé en ce que l'on isomérise le 1,7-octadiène-3-ol en 2,7-octadiène-1-ol, ou le but-1-ène-3-ol en but-2-ène-1-ol.
